(19) Europäisches Patentamt
European Patent Office
Office européen des brevets

(11) Veröffentlichungsnummer: **0 000 537**
**B1**

(12) **EUROPÄISCHE PATENTSCHRIFT**

(21) Anmeldenummer: **78100424.7**

(22) Anmeldetag: **18.07.78**

(51) Int. Cl.³: **A 01 N 43/30,**
C 07 D 317/68

(54) **Insektizide und akarizide Mittel, deren Verwendung und Verfahren zu deren Herstellung**

(30) Priorität: **28.07.77 DE 2734108**

(43) Veröffentlichungstag der Anmeldung:
**07.02.79 Patentblatt 79/03**

(45) Bekanntmachung des Hinweises auf die Patenterteilung:
**10.12.80 Patentblatt 80/25**

(84) Benannte Vertragsstaaten:
**BE CH DE FR GB NL SE**

(56) Entgegenhaltungen:
**FR - A - 1 441 954**
**NL - A - 71 12508**

(73) Patentinhaber: **BAYER AG**
**Zentralbereich Patente, Marken und Lizenzen**
**D - 5090 Leverkusen 1, Bayerwerk (DE)**

(72) Erfinder: **Mues, Volker, Dr.**
**Maréestrasse 61**
**D - 5600 Wuppertal 1 (DE)**
**Behrenz, Wolfgang, Dr.**
**Untergruendemich 14**
**D - 5063 Overath (DE)**

EP 0 000 537 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Insektizide und akarizide Mittel, deren verwendung und verfahren zu deren Herstellung

Die vorliegende Erfindung betrifft neue insektizide und akarizide synergistische Wirkstoffkombinationen aus teilweise bekannten Benzodioxolen und anderen bekannten pestiziden, insbesondere insektiziden, Wirkstoffen.

Weiterhin ist es bereits bekannt, daß die folgenden Wirkstoffe bzw. Wirkstoffgruppen pestizide, insbesondere insektizide und akarizide, Eigenschaften aufweisen:

A) Carbamate, wie z.B. das 2 - iso - Propoxy - phenyl - N - methyl - carbamat, 3,4,5 - Trimethyl - phenyl - N - methyl - carbamat, 1-Naphthyl - N - methyl - carbamat, 2,3 - Dihydro - 2,2 - dimethyl - 7 - benzofuranyl - N - methylcarbamat, 2 - [1,3 - Dioxolan(2)yl - phenyl] - N - methyl - carbamat und 2,2-Dimethyl - 1,3 - benzodioxol(4)yl - N - methyl - carbamat,

B) Carbonsäureester, wie z.B. 2,3,4,5 - Tetrahydrophthalimidomethyl - chrysanthemat und (5 - Benzyl - furyl(3)) - methyl - 2,2 - dimethyl - 3 - (2 - methylpropenyl) - cyclopropancarboxylat,

C) Phosphorsäureester, wei z.B. 0,0-Dimethyl-0-(2,2-dichlorvinyl)-phosphorsäureester, und

D) Halogenalkane, wie z.B. 1,1,1-Trichlor-2,2-bis-(4-methoxy-phenyl)-äthan und 1,1,1-Trichlor-2,2-bis-(4-chlorphenyl)-äthan.

Weiterhin sind synergistische Mischungen von Carbamaten, z.B. 2-iso-Propoxy-phenyl-N-methylcarbamat oder von Phosphorsäureestern, z.B. 0,0-Diäthyl-0-[2-isopropyl-4-methylpyrimidinyl (6)]-thionophosphorsäureester oder von natürlichen oder synthetischen Pyrethroiden mit Piperonyläthern, wie z.B. $\alpha$-[2-(2-Butoxy-äthoxy)-äthoxy]-4,5-methylendioxy-2-propyl-toluol, bekannt (vgl. Bull.Org. Health Org. 1966, *35*, Seiten 691—708; Schrader, G.: Die Entwicklung neuer insektizider Phosphorsäureester 1963, S. 158; Perkov, W.: Die Insektizide, 1966, Seiten 516—524). Doch ist die Wirksamkeit dieser synergistischen Wirkstoffkombinationen nicht befriedigend. Eine gewisse praktische Bedeutung hat bisher nur Das $\alpha$-[2-(2-Butoxy-äthoxy)-äthoxy-4,5-methylendioxy-2-propyl-toluol erlangt.

In der FR—PS 1.441.954 werden 3,4-Methylendioxobenzolnitrile als Synergisten für insektizide Carbamate und Phosphorsäureester genannt, die jedoch in ihrer Wirkung ebenso wie die obengenannten Mischungen unbefriedigend sind.

Es wurde nur gefunden, daß neue Wirkstoffkombinationen aus Benzodioxol-Derivaten der Formel (I)

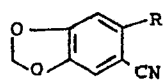

(I)

in welcher

R für Alkyl, Alkenyl, Alkinyl, Aryl oder Aralkyl steht, und

A) Carbamaten und/oder

B) Carbonsäureestern, einschließlich der natürlichen sowie synthetischen Pyrethroide, und/oder

C) Phosphorsäureestern und/oder

D) Halogenalkanen

eine besonders hohe insektizide und akarizide Wirkung aufweisen.

Die synergistische Wirkung der Verbindungen der allgemeinen Formel (I) zeigt sich bevorzugt bei den

A) Carbamaten der allgemeinen Formel (II)

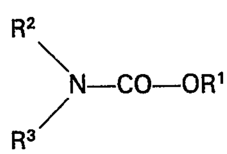

(II)

in welcher

$R^1$ für Aryl, Heterocyclus oder einen Oximrest steht,

$R^2$ für Wasserstoff oder Alkyl mit 1 bis 4 Kohlenstoffatomen steht und

$R^3$ für Alkyl, Alkylcarbonyl mit 1 bis 6 Kohlenstoffatomen im Alkylrest, der gegebenenfalls auch durch Hydroxy oder Methylthio substituiert sein kann, oder den Rest —S—Z steht, wobei

Z für einen gegebenenfalls durch Halogen substituierten aliphatischen Rest mit 1 bis 4 Kohlenstoffatomen, insbesondere $CCl_3$ und $CF_3$ sowie für gegebenenfalls bevorzugt durch Nitril, Halogen, insbesondere Chlor, Methyl, Trihalogenmethyl, Trifluormethylmercapto oder Nitro substituierten Arylrest, insbesondere Phenyl, oder für Methoxycarbonyl oder für den Rest

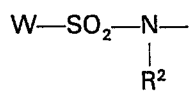

steht, wobei W für Alkyl, Halogenoalkyl oder Alkylamino, Dialkylamino oder einen gegebenenfalls bevorzugt durch Halogen, Trihalogenmethyl, Nitril, Methyl oder Nitro substituierten Arylrest steht.

Besonders bevorzugt sind Carbamate in denen

$R^1$ für Phenyl oder Naphthyl steht, die gegebenenfalls substituiert sind durch Alkyl, Alkenyl, Alkoxy, Alkylmercapto oder Alkylthioalkylen mit jeweils 1 bis 5 Kohlenstoffatomen, Dialkylamino, Dialkenylamino mit bis zu 3 Kohlenstoffatomen je Alkyl-bzw. Alkenylteil, Halogen, insbesondere Chlor, Dioxolanyl oder den Rest —N=CH—N($C_{1-4}$-Alkyl)$_2$ steht.

Weiterhin sind besonders bevorzugt Carbamate, in denen

$R^1$ für 2,3-Dihydrobenzofuranyl, Benzodioxol, Benzothienyl, Pyrimidinyl oder Pyrazolyl steht, die gegebenenfalls durch $C_{1-4}$-Alkyl, insbesondere Methyl, oder Dialkylamino mit 1 bis 4 Kohlenstoffatomen je Alkylteil substituiert sind.

Weiterhin sind besonders bevorzugt Carbamate, in denen $R^1$ für einen Oximrest der allgemeinen Formel (II a)

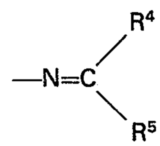

(II a)

steht, in welcher

$R^4$ und $R^5$ gleich oder verschieden sind und für Alkyl, Cycloalkyl, Alkenyl, Alkinyl, Alkoxy, Alkylmercapto, Alkoxycarbonyl, Carbonylamid, Alkylmercaptoalkyl, mit jeweils bis zu 5 Kohlenstoffatomen, Nitril, Aryl, insbesondere Phenyl, einen gegebenenfalls substituierten heterocyclischen Rest oder für Alkyl, das durch einen heterocyclischen Rest substituiert ist oder gemeinsam einen gegebenenfalls durch $C_{1-4}$-Alkyl substituierten Dioxolanyl oder Dithiolanylrest stehen;

B) Carbonsäureestern der allgemeinen Formel (III)

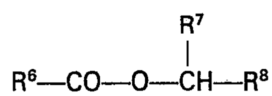

(III)

in welcher

$R^6$ für Alkyl, Aralkyl, Aryl oder Cycloalkyl steht, die gegebenenfalls substituiert sein können und

$R^7$ für Wasserstoff, Alkyl, Halogenalkyl, Alkenyl, Alkinyl, Nitril steht und

$R^8$ für Aryl oder einen Heterocyclus steht, oder gemeinsam mit $R^7$ einen gegebenenfalls substituierten Cyclopentenonring bildet.

Besonders bevorzugt sind Carbonsäureester, in denen $R^6$ für Alkyl mit 1 bis 6 Kohlenstoffatomen, das gegebenenfalls durch gegebenenfalls halogensubstituiertes Phenyl substituiert ist, Cyclopropyl, das gegebenenfalls ducrh Alkyl, Alkenyl, Halogenalkyl oder Halogenalkenyl mit jeweils bis zu 6 Kohlenstoffatomen substituiert ist, oder für Phenyl, das gegebenenfalls durch Halogen substituiert ist, steht. Bevorzugt sind Carbonsäureester, in denen $R^7$ für Wasserstoff, Alkyl mit 1 bis 6 Kohlenstoffatomen, Halogenalkyl mit 1 bis 4 Kohlenstoffatomen und bis zu 3 Halogenatomen, Nitril oder Äthinyl steht.

Weiter sind besonders bevorzugt Carbonsäureester, in denen $R^8$ für Phenyl steht, das gegebenenfalls durch $C_{1-4}$-Alkyl, Halogen, insbesondere Fluor oder Chlor, gegebenenfalls halogen- oder methylsubstituiertes Phenoxy, gegebenenfalls substituiertes Benzyl substituiert ist, ferner für Furanyl, Tetrahydrophthalimido, Benzodioxol, die gegenbenenfalls durch Halogen, insbesondere Chlor, Alkyl oder Alkenyl mit bis zu 4-Kohlenstoffatomen oder Benzyl substituiert sind, steht, und ferner für Cyclopentonon steht, das gegebenenfalls durch $C_{1-4}$-Alkyl, Furfuryl, $C_{1-5}$-Alkenyl substituiert ist.

Weiter sind besonders bevorzugt die natürlich vorkommenden Pyrethroide;

C) Phosphorsäureester der allgemeinen Formel (IV)

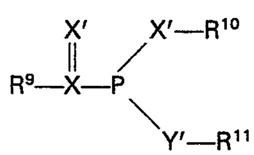

(IV)

In welcher

X′ unabhängig voneinander für O oder S steht und

Y′ für O, S, —NH— oder für eine direkte Bindung zwischen dem zentralen P-Atom und dem $R^{11}$ steht und

$R^9$ und $R^{10}$ gleich oder verschieden sind und für gegebenenfalls substituiertes Alkyl oder Aryl stehen,

$R^{11}$ für gegebenenfalls substiutiertes Alkyl, Aryl, Heteroaryl, Aralkyl, Alkenyl, Dioxanyl, oder einen

Oximrest oder für den gleichen Rest steht, an den es gebunden ist.

Besonders bevorzugt sind Phosphorsäureester, in denen

$R^9$ und $R^{10}$ gleich oder verschieden sind und für $C_{1-4}$-Alkyl oder Phenyl stehen,

$R^{11}$ für Alkyl mit 1 bis 4 Kohlenstoffatomen steht, das gegebenenfalls durch Halogen, Hydroxyl, Nitril, gegebenfalls halogensubstituiertes Phenyl, Carbonylamid, Sulfonylalkyl, Sulfoxyalkyl, Carbonylalkyl, Alkoxy, Alkylmercapto, Alkoxycarbonyl, substituiert ist, für Alkenyl mit bis zu 4 Kohlenstoffatomen, das gegebenenfalls durch Halogen, gegebenenfalls halogensubstituiertes Phenyl oder Alkoxycarbonyl substituiert ist, oder für den Oximrest der allgemeinen Formel (II a)

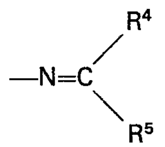

(II a)

wobei $R^4$ und $R^5$ die oben angegebene Bedeutung besitzen, insbesondere jedoch für Cyan oder Phenyl stehen,

$R^{11}$ steht ferner für Dioxanyl, das durch denselben Rest substituiert ist, an den $R^{11}$ gebunden ist, oder $R^{11}$ steht für den gleichen Rest, an den er gebunden ist, oder $R^{11}$ steht für Phenyl, das gegebenenfalls durch Methyl, Nitro, Nitril, Halogen, Methylthio substituiert ist; $R^{11}$ steht außerdem besonders bevorzugt für gegebenenfalls durch $C_{1-4}$-Alkyl, Halogen substituiert Heteroaromaten, wie Pyridin, Chinolin, Chinoxalin, Pyrimidin, Diazinon, Benzo-1,2,4-triazin;

D) Halogenalkane der Formel (V)

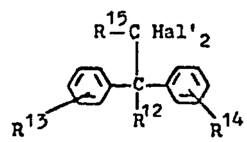

(V)

in welcher

Hal' für Chlor oder Brom

$R^{12}$ für Wasserstoff oder Hydroxyl,

$R^{13}$ und $R^{14}$ gleich oder verschieden sind und für Halogen, Alkyl oder Alkoxy und

$R^{15}$ für Wasserstoff oder Halogen stehen.

Besonders bevorzugt sind Halogenalkane, in denen

$R^{12}$ Wasserstoff oder Hydroxyl bedeutet,

$R^{13}$ und $R^{14}$ für gleiches Halogen, Alkyl bzw. Alkoxy mit 1 bis 4 Kohlenstoffatomen je Alkyl- bzw. Alkoxyrest stehen und

$R^{15}$ Halogen bedeutet.

Überraschenderweise ist die insektizide und/oder akarizide Wirkung der erfindungsgemäßen Wirkstoffkombinationen wesentlich höher als die Wirkung der Einzelkomponenten bzw. die Summe der Wirkungen der Einzelkomponenten. Sie ist ferner wesentlich höher als die Wirkung der bereits bekannten Wirkstoffkombination aus 2-iso-Propoxy-phenyl-N-methyl-carbamat und Piperonylbutoxyd. Außerdem zeigen die erfindungsgemäß verwendbaren Benzodioxol-Derivate ausgezeichnete synergistische Wirkamkeit nicht nur bei einer Wirkstoffklasse, sondern bei Wirkstoffen aus den verschiedensten chemischen Stoffgruppen.

Somit stellen die erfindungsgemäßen Benzodioxol-Derivate enthaltenden synergistischen Mischungen eine wertvolle Bereicherung der Technik dar.

Die für die erfindungsgemäße Kombination zu verwendenden Synergisten sind durch die Formel (I) allgemein definiert, Vorzugsweise stehen in der Formel (I) jedoch:

R für geradkettiges oder verzweigtes Alkyl mit 1 bis 8 Kohlenstoffatomen, insbesondere 1 bis 5 Kohlenstoffatomen, oder für geradkettiges oder verzweigtes Alkenyl mit 2 bis 8 Kohlenstoffatomen, insbesondere mit 2 bis 5 Kohlenstoffatomen, oder für geradkettiges oder verzweigtes Alkinyl mit 2 bis 8 Kohlenstoffatomen, insbesondere mit 2 bis 5 Kohlenstoffatomen, oder für Phenyl oder Benzyl.

Als Beispiele für die erfindungsgemäß verwendbaren Benzodioxol-Derivate der Formel (I) seien im einzelnen genannt:

6-Methyl-, 6-Äthyl-, 6-n-Propyl-, 6-iso-Propyl-, 6-n-Butyl-, 6-iso-Butyl-, 6-sec.-Butyl-, 6-tert.-Butyl-, 6-n-Pentyl-, 6-iso-Pentyl-, 6-tert.-Pentyl-, 6-Vinyl-, 6-Allyl-, 6-Propenyl-, 6-Methallyl-, 6-Crotonyl-, 6-Buten-1'-yl-, 6-Buten-3'-yl-, 6-Äthinyl-, 6-Propin-1'-yl-, 6-Propargyl-, 6-Phenyl-, 6-Benzyl-3.4-methylendioxy-benzonitril.

Diese Verbindungen sind teilweise neu, können aber nach literaturbekannten Verfahren hergestellt werden (vgl.z.B. Pailer, Schleppnik, Monatshefte für Chemie *96*, 1554—1562; ebda. *98*, 1603—1612).

Das folgende Formelschema illustriert den Reaktionsverlauf:

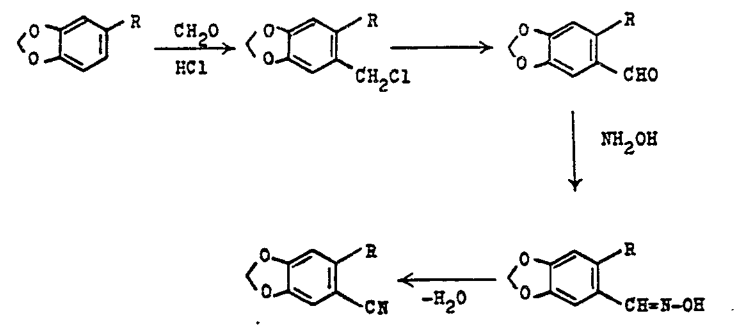


Für den Fall, daß R für n-Propyl steht, verläuft die Synthese über folgende Stufen: Safrol wird zum Dihydrosafrol hydriert und der Chlormethylierung zum 6-Chlormethyl-dihydrosafrol unterworfen; Reaktion mit Urotropin nach Sommelet führt zum 6-n-Propyl-piperonal. Dessen Aldoxim wird mit Acetanhydrid zum 3.4-Methylendioxy-6-n-propyl-benzonitril dehydratisiert.

Die einzelnen Stufen der angegebenen Synthesewege sind bekannt oder erfolgen nach an sich bekannten Verfahrensweisen. Beispielsweise kann die Herstellung der jeweils als Zwischenprodukte dienenden 6-Chlormethylverbindungen nach den in den US-Patentschriften 2 485 600 und 2 485 680 angegebenen Verfahren erfolgen.

Zu den als Mischkomponenten zu verwendenden Carbamaten (Gruppe A) der Formel (II) gehören: 2-Methylphenyl-, 2-Äthyphenyl-, 2-n-Propylphenyl-, 2-Methoxyphenyl-, 2-Äthoxyphenyl-, 2-iso-Propoxyphenyl-, 4-Methylphenyl-, 4-Athylphenyl-, 4-n-Propylphenyl-, 4-Methoxyphenyl-, 4-Äthoxyphenyl-, 4-n-Propoxyphenyl-, 3,4,5-Trimethylphenyl-, 1-Naphthyl-, 2 3-Dihydro-2,2-dimethyl-7-benzofuranyl-, 2-[1,3-Dioxolan(2)yl-phenyl]- bzw. 2,2-Dimethyl-1,3-benzodioxol(4)yl-N-methyl-carbamat und die entsprechenden -N-methyl-N-acetyl-, -N-methyl-N-trifluormethylthio-, -N-methyl-N-dichlormonofluormethylthio-bzw. -N-methyl-N-dimethylaminothiocarbamate.

Diese Verbindungen, ihre Herstellung und ihre Verwendung sind bekannt (vergleiche z.B. US Patentschriften 3 009 855; 2 903 478 und 3 111 539).

Zu den weiterhin als Mischungskomponenten gegebenenfalls zu verwendenden Carbonsäureestern (Gruppe B) der Formel (III) geheren:
Essigsäure - [1 - (3,4 - dichlorphenyl) - 2,2,2-trichloräthyl] - ester, 2,3,4,5 - Tetrahydrophthalimidomethylchrysanthemat und (5 - Benzylfuryl(3)) - methyl - 2,2 - dimethyl - 3 - (2 - methylpropenyl) - cyclopropancarboxylat. Die aufgezählten Verbindungen sind bekannt und größtenteils allgemein bekannte Handelsprodukte [vergleiche R. Wegler "Chemie der Pflanzenschutz- und Schädlingsbekämpfungsmittel" Bd. 1; Seiten 87—118, Heidelberg (1970)].

Zu den weiterhin als Mischungskomponenten gegebenenfalls zu verwendenden Phosphorsäureestern der Formel (IV) gehören:
0,0-Dimethyl- bzw. 0,0-Diäthyl-0-(2,2-dichlor- bzw. 2,2-dibromvinyl)-phosphorsäureester,
0,0 - Diäthyl - 0 - (4 - nitro - phenyl)- thionophosphorsäureester,
0,0 - Dimethyl - 0 - (3-methyl - 4 - methylthio) - thionophosphorsäureester,
0,0 - Dimethyl - 0 - (3 - methyl - 4 - nitro) - thionophosphorsäureester,
0 - Äthyl - S - n - propyl - 0 - (2,4 - dichlorphenyl) - thionophosphorsäureester,
0 - Äthyl - S - n - propyl - 0 - (4 - methylthio - phenyl) - thionophosphorsäureester,
0,0 - Dimethyl - S - [4 - oxo - 1,2,3 - benzotriazin(3)yl - methyl] - thionothiolphosphorsäureester,
0 - Methyl - 0 - [2 - iso - propyl - 6 - methoxy - pyrimidin(4)yl] - thionomethanphosphonsäureester,
0,0 - Diäthyl - 0 - [2 - iso - propyl - 6 - methyl - pyrimidin(4)yl] - thionophosphorsäureester,
0,0 - Diäthyl - 0 - [3 - chlor - 4 - methyl - cumarin(7)yl] - thionophosphorsäureester,
0,0 - Dimethyl - 2,2,2 - trichlo - 1 - hydroxy - äthan - phosphonsäureester,
0,0 - Dimethyl - S - (methylcarbamoylmethyl) - thionophosphorsäureester.

Die Verbindungen der Formel (IV) sind bekannt und nach literaturbekannten Verfahren gut zugänglich (vergleiche zum Beispiel US-Patentschrift 2 956 073, Deutsche Auslegeschrift 1 167 324, Belgische Patentschrift 633 478).

Zu den weiterhin als Mischungskomponenten gegebenenfalls zu verwendenden Halogenalkanen (Gruppe D) der Formel (V) gehören:
1,1,1 - Trichlor - 2,2 - bis - (4 - chlor- bzw. 4 - methoxyphenyl) - äthan, 1,1,1 - Trichlor - 2 - hydroxy - 2,2 - bis - (4 - chlorphenyl) - äthan und 1,1 - Dichlor - 2,2 - bis - (4 - äthylphenyl) - äthan.
Diese Verbindungen, ihre Herstellung und ihre Verwendung sind bekannt (vergleiche zum Beispiel US-Patentschriften 2 420 928, 2 464 600, 2 883 428 und 2 917 553).

Wie bereits erwähnt, zeigen die neuen Wirkstoffkombinationen der erfindungsgemäß verwendbaren Benzodioxol-Derivate der Formel (I) mit Carbamaten, Carbonsäureestern, Phosphorsäureestern und/oder Halogenalkanen eine hervorragende Wirkungssteigerung gegenüber den Einzelwirkstoffen bzw. gegenüber deren Summe.

Die Gewichtsverhältnisse der Wirkstoffgruppen können dabei in relativ großen Bereichen schwanken. Im allgemeinen werden die Benzodioxolderivate mit den übrigen Wirkstoffen im Verhältnis 0,1:10 bis 10:0,1 eingesetzt. Besonders geeignet haben sich jedoch Mischungsverhältnisse von 0,5:1,0 bis 3,0:1,0 erwiesen. Die erfindungsgemäßen Wirkstoffkombinationen bewirken nicht nur eine schnelle knock-down-Wirkung, sondern bewirken auch die nachhaltige Abtötung aller oder einzelner Entwicklungsstadien tierischer Schädlinge, insbesondere Insekten. Zu den Schädlingen gehören diejenigen, die in die Landwirtschaft, in Forsten, im Vorrats- und Materialschutz sowie auf dem Hygienesektor vorkommen. Dazu gehören:
Aus der Ordnung der Isopoda z. B. Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z. B. Forficula auricularia.
Aus der Ordnung der Isoptera z. B. Reticulitermes spp.,
Aus der Ordnung der Anoplura z. B. Phylloxera vastatrix, Pemphiqus spp., Pediculus humanus corporis, Haematopinus spp., Linognathus spp.
Aus der Ordnung der Mallophaga z. B. Trichodectes spp., Damalinea spp.
Aus der Ordnung der Thysanoptera z. B. Hercinothrips femoralis, Thrips tabaci.
Aus der Ordnung der Heteroptera z. B. Eurygaster spp., Dysdercus intermedius, Piesma quadrata, Cimex lectularius, Rhodnius prolixus, Triatoma spp.
Aus der Ordnung der Homoptera z. B. Aleurodes brassicae, Bemisia tabaci, Trialeurodes vaporariorum, Aphis gossypii, Brevicoryne brassicae, Cryptomycus ribis, Doralis fabae, Doralis pomi, Ertosoma lanigerum, Hyalopterus arundinis, Macrosiphum evenae, Myzus spp., Phorodon humuli, Rhopalosiphum padi, Empoasca spp., Euscelis bilobatus, Nephotettix cincticeps Lecanium corni, Saissetia oleae, Laodelphax striatellus, Nilaparvata lugens, Aonidiella aurantii, Aspidiotus hederae, Pseudococcus spp., Psylla spp..

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp.,Euxoa spp., Feltia spp., Earias insulana, Heliothis spp., Laphygma exigua, Mamestra brassicae, Panolis flammea, Prodenia litura, Spondoptera spp., Trichoplusia ni, Carpocapaa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Paylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilia, Hypera postica, Dermestes spp., Trogoderma spp., Anthrenus spp., Attagenus spp., Lyctus spp., Meligethes seneus, Ptinus spp., Niptus hololeucua, Gibbium paylloides, Tribolium spp., Tenebrio molitor, Agriotes spp., Conoderus spp., Melolontha melolontha, Amphimallon solstitialis, Costelytra zealandica.
Aus der Ordnung der Hymenoptera z. B. Diprion spp., Hoplocampa spp., Lasius spp., Monomorium pharaonis, Vespa spp.
Aus der Ordnung der Diptera z. B. Aëdes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z. B. Xenopsylla cheopis, Ceratophyllus spp.
Aus der Ordnung der Arachnida z. B. Scorpio maurus, Latrodectus mactans.
Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffkombinationen können in die üblichen Formulierungen übergeführt werden, wie Lösungen, Emulsionen, Spritzpulver, Suspensionen, Pulver, Staubemittel, Schäume, Pasten, lösliche Pulver, Granulate, Aerosole, Suspensions-Emulsionskonzentrate, Saatgutpucer, Wirkstoff-imprägnierte Natur- und synthetische Stoffe, Feinstverkapselungen in polymeren Stoffen und in Hüllmassen für Saatgut, ferner in Formulierungen mit Brennsätzen, wie Raucherpatronen, -dosen, -spiralen u.a. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Tragerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslosungsmittel verwendet werden. Als flüssige Losungsmittel kommen im wesentlichen infrage: Aromaten, wie Xylol, Toluol, oder Alkylnaphthaline, chlorierte Aromaten oder chlorierte aliphatische Kuhlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie ceren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temparatur und unter Normaldruck gasförmig sind, z.B. Aerosol-Treibgase, wie Halogenkohlenwasserstoffe sowie Butan, Propan, Stickstoff und Kohlendioxid; als fest Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktioniert natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z. B. Lignin—Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugswise zwischen 0,5 und 90%.

Die Anwendung der erfindungsgemäßen Wirkstoffkombinationen erfolgt in Form ihrer handelsüblichen Formulierungen und/oder den aus diesen Formulierungen bereiteten Anwendungsformen.

Der gesamte Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffkombinationen durch eine hervorragende Residualwirkung auf Holz und Ton sowie durch eine gute Alkalistabilität auf gekälkten Unterlagen aus.

Die folgenden Beispiele zeigen die synergistischen Eigenschaften der erfindungsgamäß verwendbaren Benzodioxol-Derivate bei folgenden bekannten Wirkstoffen:

(A) 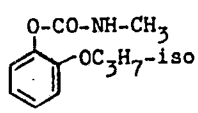

(B) 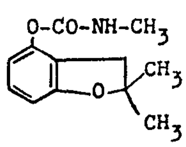

(C) 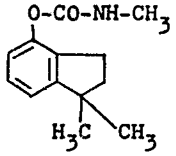

(D) 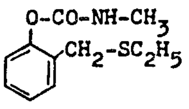

(E) 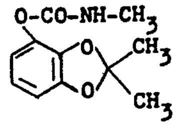

(F) 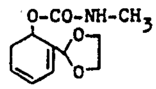

(G) 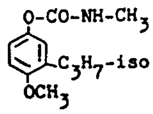

(H) 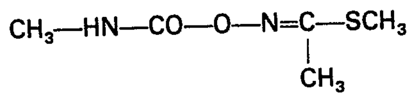

(I) Pyrethrine als 25%iger Extrakt

(K) 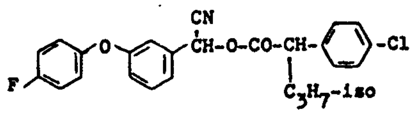

(L) 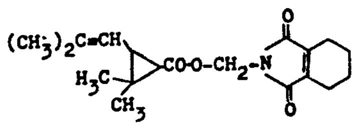

(M) 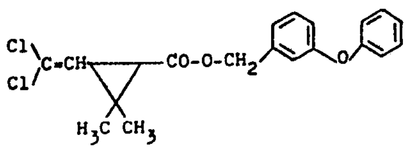

(N) 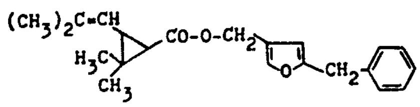

(O) 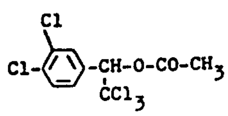

(P) 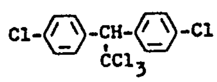

(Q) 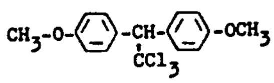

(R) 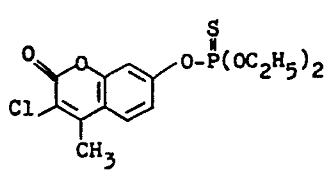

(S) 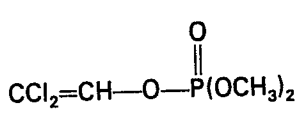

(T) 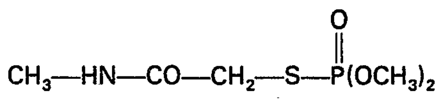

(U) 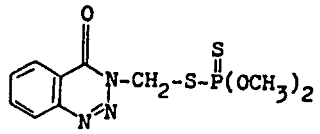

(V) 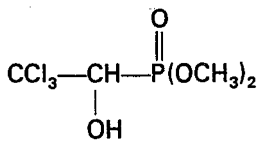

Zu Vergleichszwecken wurde zusätzlich das bekannte ebenfalls als Syaergist verwendete Piperonylbutoxid verwendet:

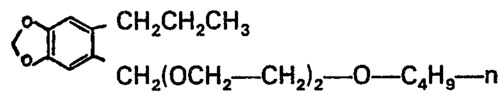

In den Tabellen der folgenden Beispiele wurden die bekannten Wirkstoffe sowie der bekannte Synergist mit den vorstehend angegebenen Großbuchstaben gekennzeichnet, während für die erfindungsgemäß verwendbaren Synergisten die bei den Herstellungsbeispielen angegebenen Ziffern verwendet werden.

## Beispiel A

$LT_{100}$ -Test
Testtiere: Phosphorsäureesterresistente Musca domestica (Stamm Weymanns)
Lösungsmittel: Aceton

Von den Wirkstoffen, Synergisten und Gemischen aus Wirkstoffen und Synergisten werden Lösungen hergestellt und 2,5 ml davon in Petrischalen auf Filterpapierscheiben von 9,5 cm Durchmesser pipetiert. Das Filterpapier saugt die Lösungen auf. Die Petrischalen bleiben so lange offen stehen, bis das Lösungsmittel vollstängid verdunstet ist. Anschließend gibt man 25 Testtiere in die Petrischalen und bedeckt sie mit einem Glasdeckel.

Der Zustand der Testtiere wird bis zu 6 Stunden laufend kontrolliert. Es wird diejenige Zeit ermittelt, die für eine 100 %ige knock down-Wirkung erforderlich ist. Wird die $LT_{100}$ nach 6 Stunden nicht erreicht, wird der % Saltz der knock down gegangenen Testtiere festgestellt.

Konzentrationen der Wirkstoffe, Synergisten und Gemische und ihre Wirkungen gehen aus der nachfolgenden Tabelle hervor.

TABELLE

LT 100–Test mit phosphorsäureester-resistenten Musca domestica (Stamm Weymanns)

| Wirkstoffe Kennbuchstabe | bzw. Synergisten ( ) Beispiel Nr. | Konzentrationen in % | LT 100 nach Minuten |
|---|---|---|---|
| A | | 1,0 | 360' = 0 % |
| B | | 1,0 | 360' = 60 % |
| C | | 1,0 | 360' = 0 % |
| D | | 1,0 | 360' = 20 % |
| E | | 1,0 | 360' = 0 % |
| F | | 1,0 | 360' = 0 % |
| G | | 1,0 | 360' = 15 % |
| H | | 0,04 | 360' = 0 % |
| I | | 0,04 | 360' = 60 % |
| K | | 0,04 | 240' |
| L | | 0,04 | 360' = 45 % |
| M | | 0,008 | 360' = 95 % |
| N | | 0,008 | 360' = 0 % |
| O | | 1,0 | 360' = 10 % |
| P | | 1,0 | 360' = 5 % |
| Q | | 1,0 | 360' = 20 % |
| R | | 1,0 | 360' = 0 % |
| S | | 0,008 | 360' = 90 % |
| T | | 0,04 | 360' = 25 % |
| U | | 1,0 | 360' = 65 % |
| V | | 1,0 | 360' = 45 % |
| | Piperonylbutoxid | 1,0 | 360' = 0 % |
| (1) | | 1,0 | 360' = 70 % |
| (2) | | 1,0 | 360' = 0 % |
| (3) | | 1,0 | 360' = 60 % |
| (4) | | 1,0 | 360' = 60 % |

| Wirkstoffe Kennbuchstabe | bzw. Synergisten Beispiel Nr. | Konzentrationen in % | LT 100 nach Minuten |
|---|---|---|---|
| A + | Piperonylbutoxid | 0,2 + 0,2 | 360' = 85 % |
| A + | 1 | 0,008 + 0,008 | 240' |
| A + | 2 | 0,008 + 0,008 | 180' |
| A + | 3 | 0,008 + 0,008 | 150' |
| B + | Piperonylbutoxid | 0,2 + 0,2 | 360' = 95 % |
| B + | 1 | 0,008 + 0,008 | 180' |
| B + | 2 | 0,008 + 0,008 | 210' |
| B + | 3 | 0,04 + 0,04 | 90' |
| C + | Piperonylbutoxid | 1,0 + 1,0 | 360' = 80 % |
| C + | 1 | 0,2 + 0,2 | 360' |
| C + | 2 | 0,04 + 0,04 | 360' |
| C + | 3 | 0,04 + 0,04 | 360' |
| D + | Piperonylbutoxid | 0,2 + 0,2 | 360' = 0 % |
| D + | 1 | 0,04 + 0,04 | 360' |
| D + | 2 | 0,04 + 0,04 | 360' = 85 % |
| D + | 3 | 0,04 + 0,04 | 360' |
| E + | Piperonylbutoxid | 0,2 + 0,2 | 360' = 75 % |
| E + | 1 | 0,008 + 0,008 | 240' |
| E + | 2 | 0,008 + 0,008 | 210' |
| E + | 3 | 0,008 + 0,008 | 180' |
| F + | Piperonylbutoxid | 1,0 + 1,0 | 360' = 90 % |
| F + | 1 | 0,2 + 0,2 | 150' |
| F + | 2 | 0,2 + 0,2 | 180' |
| F + | 3 | 0,2 + 0,2 | 180' |
| G + | Piperonylbutoxid | 1,0 + 1,0 | 360' = 90 % |
| G + | 1 | 0,04 + 0,04 | 120' |
| G + | 2 | 0,04 + 0,04 | 150' |
| H + | Piperonylbutoxid | 1,0 + 1,0 | 90' |
| H + | 1 | 1,0 + 1,0 | 60' |
| H + | 2 | 1,0 + 1,0 | 60' |
| J + | Piperonylbutoxid | 0,04 + 0,04 | 90' |
| J + | 1 | 0,04 + 0,04 | 75' |
| J + | 2 | 0,04 + 0,04 | 45' |
| K + | Piperonylbutoxid | 1,0 + 1,0 | 60' |
| K + | 1 | 1,0 + 1,0 | 30' |
| K + | 2 | 0,2 + 0,2 | 60' |
| L + | Piperonylbutoxid | 0,04 + 0,04 | 360' |
| L + | 1 | 0,04 + 0,04 | 120' |
| L + | 2 | 0,04 + 0,04 | 75' |

| Wirkstoffe Kennbuchstabe | bzw. Synergisten Beispiel Nr. | Konzentrationen in % | LT 100 nach Minuten |
|---|---|---|---|
| M + | 1 | 0,008 + 0,008 | 90' |
| M + | 2 | 0,008 + 0,008 | 105' |
| N + | Piperonylbutoxid | 0,04 + 0,04 | 90' |
| N + | 1 | 0,04 + 0,04 | 45' |
| N + | 2 | 0,04 + 0,04 | 45' |
| O + | Piperonylbutoxid | 0,04 + 0,04 | 360' = 15 % |
| O + | 1 | 0,04 + 0,04 | 360' |
| O + | 2 | 0,04 + 0,04 | 360' = 70 % |
| P + | Piperonylbutoxid | 1,0 + 1,0 | 360' = 20 % |
| P + | 1 | 0,2 + 0,2 | 210' |
| Q + | Piperonylbutoxid | 1,0 + 1,0 | 360' = 85 % |
| Q + | 1 | 1,0 + 1,0 | 90' |
| Q + | 2 | 1,0 + 1,0 | 210' |
| Q + | 3 | 1,0 + 1,0 | 180' |
| R + | Piperonylbutoxid | 1,0 + 1,0 | 360' = 5 % |
| R + | 1 | 1,0 + 1,0 | 360' = 95 % |
| R + | 3 | 1,0 + 1,0 | 360' |
| S + | Piperonylbutoxid | 0,2 + 0,2 | 75' |
| S + | 1 | 0,2 + 0,2 | 45' |
| S + | 2 | 0,2 + 0,2 | 30' |
| S + | 3 | 0,2 + 0,2 | 30' |
| T + | Piperonylbutoxid | 0,04 + 0,04 | 360' = 60 % |
| T + | 1 | 0,04 + 0,04 | 150' |
| T + | 2 | 0,04 + 0,04 | 120' |
| T + | 3 | 0,04 + 0,04 | 150' |
| U + | Piperonylbutoxid | 1,0 + 1,0 | 210' |
| U + | 1 | 1,0 + 1,0 | 60' |
| U + | 3 | 1,0 + 1,0 | 75' |
| V + | Piperonylbutoxid | 1,0 + 1,0 | 360' = 90 % |
| V + | 1 | 1,0 + 1,0 | 240' |
| V + | 3 | 1,0 + 1,0 | 240' |

## Herstellungsbeispiel

a) 6-Chlormethyl-dihydrosafrol

164,2g (1,0 Mol) Dihydrosafrol werden mit 15 og 40%igem Formalin und 500 g konzentrierter Salzsäure 36 Stunden bei 20—30°C gerührt. Anschließend verdünnt man die Reaktionsmischung mit Wasser, extrahiert diese mit Toluol und wäscht die organische Phase mit einer Natriumbicarbonatlösung. Nach dem Trocknen der Lösung über Natriumsulfat wird eingeengt und der Rückstand destilliert. Es werden 173,5g (82% der Theorie) an 6-Chlormethyl-dihydrosafrol mit dem Siedepunkt 124°C/ 3 mm Hg erhalten.

b) 6-n-Propyl-3,4-methylendioxy-benzaldehyd

Zu einer Lösung aus 212,7g (1,0 Mol) 6-Chlormethyl-dihydrosafrol in 1000 ml Methylenchlorid gibt man 154 g (1,1 Mol) Urotropin und erhitzt die Reaktionsmischung unter Rückfluß und unter Rühren 4 Stunden. Danach wird abgekühlt, der ausgefallene kristalline Rückstand abgesaugt und an der Luft getrocknet. Dieser wird dann unter Rückfluß in 1000 ml 50%iger Essigsäure 4 Stunden gerührt, auf 60°C abgekühlt, mit konz. Salzsäure auf $p_H$ 2 eingestellt und weitere 10 Minuten unter Rückfluß. Danach wird die Reaktionsmischung abgekühlt, mit Wasser verdünnt und mit Methylenchlorid extrahiert. Die organische Phase wird neutral gewaschen, getrocknet, eingeengt und schließlich der Rückstand destilliert. Es werden 137 g (71% der Theorie) an 6-n-Propyl-3,4-methylendioxybenzaldehyd mit dem Siedepunkt von 115°C/ 0,01 mm Hg gewonnen.

c) 6-n-Propyl-3,4-methylendioxy-benzaldoxim

Zu einer Suspension von 37,1 g (0,533 Mol) Hydroxylaminhydrochlorid in 500 ml Äthanol gibt man 21,3 g (0,533 Mol)

Natriumhydroxyd in 27 ml Wasser und fügt dann eine Lösung von 77,0 g (0,4 Mol) 6-n-Propyl-3,4-methylendioxy-benzaldehyd in 150 ml Äthanol zu. Nach vierstündigem Rühren unter Rückfluß kühlt man die Reaktionsmischung ab, zieht das Lösungsmittel ab und behandelt den Rückstand mit Wasser. Man saugt ab und verreibt den Rückstand mit einem Gemisch aus Petroläther/ Äther (6:1), saugt wiederum ab und trocknet an der Luft. Man erhält 72 g (87% der Theorie) an Kristallen mit dem Schmelzpunkt 83°C.

d) 6-n-Propyl-3,4-methylendioxy-benzonitril

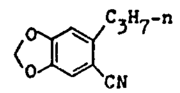

(4)

62,2 g (0,3 Mol) 6-n-Propyl-3,4-methylendioxy-benzaldoxim werden mit 400 ml Acetanhydrid 3 Stunden unter Rückfluß gerührt, die Reaktionslösung abgekühlt und in Wasser gegossen. Nach 2 stündigem Stehenlassen extrahiert man mit Toluol, wäscht die organische Phase erst mit Wasser, dann mit Natriumhydrogencarbonat-Lösung und nochmals mit Wasser neutral. Nach dem Trocknen über Natriumsulfat wird die organische Phase eingeengt und destilliert. Man erhält 38 g (67% der Theorie) mit dem Siedepunkt 116°C/3 mm Hg.

Analog können hergestellt werden:

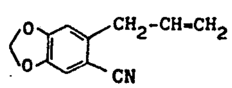

(1)

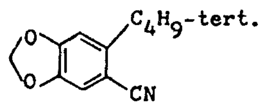

(2)

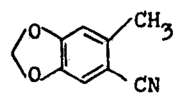

(3)

**Patentansprüche**

1. Insektizide und akarizide Mittel, gekennzeichnet durch einen Gehalt an einer Wirkstoffkombination bestehend aus Benzodioxolderivaten der allgemeinen Formel (I)

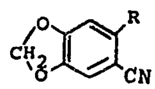

(I)

in welcher
R für Alkyl, Alkenyl, Alkinyl, Aryl oder Aralkyl steht,
und
A) Carbamaten und/oder
B) Carbonsäureestern, einschließlich der natürlichen sowie synthetischen Pyrethroide, und/oder
C) Phosphorsäureester und/oder
D) Halogenalkanen.

2. Insektizide und akarizide Mittel gemäß Anspruch 1, dadurch gekennzeichnet, daß in der Wirkstoffkombination das Gewichtsverhältnis von Benzodioxol-Derivaten zu Wirkstoffen zwischen 0,1:10 und 10:0,1 liegt.

3. Verwendung von Wirkstoffkombinationen gemäß Anspruch 1 oder 2 zur Bekämpfung von Insekten und Spinnentieren.

4. Verfahren zur Herstellung von insektiziden und akariziden Mitteln, dadurch gekennzeichnet, daß man eine Wirkstoffkombination gemäß Anspruch 1 oder 2 mit Streckmitteln und/oder oberflächenaktiven Stoffen vermischt.

**Claims**

1. Insecticidal and acaricidal agents, characterised in that they contain an active compound combination consisting of benzodioxole derivatives of the general formula (I)

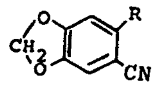

(I)

in which
R represents alkyl, alkenyl, alkinyl, aryl or aralkyl and
A) Carbamates and/or
B) Carboxylic acid esters, including the natural and synthetic pyrethroids, and/or
C) Phosphoric acid esters and/or
D) Halogenoalkanes.

2. Insecticidal and acaricidal agents according to Claim 1, characterised in that in the active compound combination the weight ratio of benzodioxole derivatives to active compounds is between 0.1:10 and 10:0.1

3. Use of active compound combinations according to Claim 1 or 2 for combating insects and arachnidae.

4. Process for the preparation of insecticidal and acaricidal agents, characterised in that an active compound combination according to Claim 1 or 2 is mixed with extenders and/or surface-active agents.

**Revendications**

1. Compositions insecticides et acaricides, caractérisées en ce qu'elles contiennent une association de substances actives constituée par des dérivés de benzodioxole de formule (I):

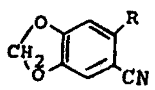

(I)

dans laquelle:
R est un groupe alkyle, alcényle, alcynyle, aryle ou aralkyle,
et par
A) des carbamates et/ou
B) des esters d'acides carboxyliques, y compris les pyréthrines naturelles ainsi que synthétiques, et/ou
C) des esters d'acide phosphorique et/ou
D) des halogênalcanes.

2. Compositions insecticides et acaricides suivant la revendication 1, caractérisées en ce que le rapport en poids des dérivés de benzodioxole aux substances actives dans l'association de substances actives se situe entre 0,1:10 et 10:0,1.

3. Utilisation d'associations de substances actives suivant la revendication 1 ou 2 dans la lutte contre des insectes et des acariens.

4. Procédé de production de compositions insecticides et acaricides, caractérisé en ce qu'on mélange une association de substances actives suivant la revendication 1 ou 2 avec des diluants et/ou des substances tensio-actives.